# EUROPÄISCHE PATENTANMELDUNG

(11) **EP 2 407 184 A2**
(43) Veröffentlichungstag der Anmeldung: **18.01.2012**
(21) Anmeldenummer: 11172366.4
(22) Anmeldetag: 01.07.2011
(51) Int. Cl.: A61L 31/02, A61L 31/10, A61L 31/14, A61L 31/16

(54) **Abluminal beschichtete Wirkstoff-freisetzende Stents mit einer formschlüssigen Schutzschicht**

(30) Priorität: 16.07.2010 US 364817 P
(71) Anmelder: Biotronik AG, 8180 Bülach (CH)
(72) Erfinder: Klocke, Björn, 8006 Zürich (CH); Harder, Claus, 91080 Uttenreuth (DE); Borck, Alexander, 91086 Aurachtal (DE)
(74) Vertreter: Lindner-Vogt, Karin L.

(57) **Zusammenfassung**

Die vorliegende Erfindung bezieht sich auf einen Wirkstoff-freisetzenden Stent mit einem Grundkörper aus einem Implantatwerkstoff, wobei der Grundkörper auf der abluminalen Seite teilweise oder ganz mit einer Wirkstoff-freisetzenden Beschichtung bedeckt ist, die ein Polymer und einen Wirkstoff mit antiproliferativer Wirkung umfasst oder daraus besteht, dadurch gekennzeichnet, dass die luminale Oberfläche und die abluminale, die Wirkstoff-freisetzende Beschichtung tragende Oberfläche des Stents von einer biokorrodierbaren Schutzschicht formschlüssig bedeckt ist.

## Beschreibung

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten ("gecrimpten") Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen einen meist röhrenförmigen Grundkörper von ausreichender Tragkraft, um das verengte Gefäß im gewünschten Maße offen zu halten, durch dessen Lumen der Blutfluss ungehindert weiterläuft. Dieser Grundkörper wird in der Regel von einer gitterartigen, aus Struts (Streben) bestehenden Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist.

Der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßer Verwendung mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Implantatwerkstoffe für Stents umfassen Polymere, metallische Werkstoffe und keramische Materialien (zum Beispiel als Beschichtung). Biokompatible Metalle und Metalllegierungen für Permanentimplantate beinhalten beispielsweise rostfreie Stähle (zum Beispiel 316L), Kobaltbasislegierungen (zum Beispiel CoCrMo-Gußlegierungen, CoCrMo-Schmiedelegierungen, CoCrWNi-Schmiedelegierungen und CoCrNiMo-Schmiedelegierungen), Reintitan und Titanlegierungen (z. B. cp Titan, TiAl6V4 oder TiAl6Nb7) und Goldlegierungen. Im Bereich biokorrodierbarer Stents wird der Einsatz von Magnesium oder Reineisen sowie biokorrodierbaren Basislegierungen der Elemente Magnesium, Eisen, Zink, Molybdän und Wolfram vorgeschlagen.

Eine biologische Reaktion auf polymere, keramische oder metallische Implantatwerkstoffe hängt von der Konzentration, Einwirkdauer und Art der Zuführung ab. Häufig führt die Gegenwart eines Implantatwerkstoffs zu Entzündungsreaktionen, deren Auslöser mechanische Reize, chemische Stoffe aber auch Stoffwechselprodukte sein können.

Ein wesentliches Problem der Stentimplantation in Blutgefäße ist die Restenose, welche beispielsweise aufgrund eines überschießenden neointimalen Wachstums eintreten kann, welches durch eine starke Proliferation der umliegenden arteriellen glatten Muskelzellen und/oder eine chronische Entzündungsreaktion hervorgerufen wird. Alternativ oder zusätzlich kann die Restenose durch die physiologische Wirkung der Freisetzung von Abbauprodukten des Stents, insbesondere des biokorrodierbaren Stents, hervorgerufen und/oder gefördert werden. So entsteht beispielsweise beim Abbau magnesiumhaltiger Stents ein basisches Milieu, welches zu einem verstärkten Muskeltonus des umliegenden Gefäßmuskels führen kann. Infolge eines solchen verstärkten Muskeltonus kann der Querschnitt des Stents abnehmen oder die Stentintegrität sogar vorzeitig verloren gehen.

Strategien zur Vermeidung der Restenose konzentrieren sich beispielsweise darauf, die Proliferation umliegender Zellen durch Medikation, z. B. Behandlung mit Wirkstoffen mit antiproliferativer Wirkung (z. B. Cytostatika), zu hemmen. Die Wirkstoffe können zum Beispiel auf der Implantatoberfläche in Form einer Beschichtung bereitgestellt werden. Wirkstoffe, die in diesem Zusammenhang bereits vorgeschlagen oder eingesetzt werden, sind Sirolimus, Derivate davon oder Taxane, wie z. B. Paclitaxel oder Salze davon.

Zur Applikation wird der Stent meist mit einer Wirkstoff-freisetzenden Beschichtung versehen, wobei sich gezeigt hat, dass Stents, die die Wirkstoff-freisetzende Beschichtung ausschließlich auf der abluminalen Seite des Stents aufweisen, solchen Stents überlegen sind, die die Wirkstoff-tragende Beschichtung auch auf der luminalen Seite tragen.

Ein Problem solcher Stents mit insbesondere nur abluminaler Wirkstoff-freisetzender Beschichtung (sogenannte "drug eluting stents" oder DES) ist, dass durch die mechanische Belastung während des Crimpens und des anschließenden Aufweitens am Zielort ein Teil der Wirkstoff-freisetzenden Beschichtung unkontrollierbar verloren geht. Dies führt einerseits dazu, dass die Menge an Wirkstoff, die tatsächlich am Zielort für die Applikation zur Verfügung steht, nur schwer vorhersagbar ist und es somit zu einer Unterdosierung kommen kann. Andererseits handelt es sich bei den verwendeten Wirkstoffen um Wirkstoffe mit erheblichem Nebenwirkungspotential, die, an einer unbeabsichtigten Stelle appliziert oder in hohem Maße systemisch abgegeben, negative Auswirkungen auf den behandelten Patienten haben können.

Aufgabe der vorliegenden Erfindung ist es, mindestens einen der Nachteile des Standes der Technik zu vermindern oder zu vermeiden.

Diese Aufgabe wird gelöst durch Bereitstellung eines Wirkstoff-freisetzenden Stents mit einem Grundkörper aus einem Implantatwerkstoff, wobei der Grundkörper auf der abluminalen Seite teilweise oder ganz mit einer Wirkstoff-freisetzenden Beschichtung bedeckt ist, die ein Polymer und einen Wirkstoff mit antiproliferativer Wirkung umfasst oder daraus besteht, dadurch gekennzeichnet, dass die luminale Oberfläche und die abluminale (die Wirkstoff-freisetzende Beschichtung tragende) Oberfläche des Stents von einer biokorrodierbaren Schutzschicht formschlüssig bedeckt ist.

Der erfindungsgemäße Stent weist die Wirkstoff-freisetzende Beschichtung bevorzugt ausschließlich auf der abluminalen Seite des Grundkörpers auf.

Der vorliegenden Erfindung liegt die Erkenntnis zugrunde, dass sich ein vorzeitiges Ablösen der Wirkstoff-tragenden Beschichtung vom Grundkörper des Stents besonders effektiv dadurch verhindern lässt, dass der gesamte Grundkörper einschließlich der Wirkstoff-tragenden Beschichtung sowohl auf der luminalen als auch auf der abluminalen Seite mit einer biokorrodierbaren Schutzschicht überzogen wird, die den Stent formschlüssig überzieht. Diese Schutzschicht führt dazu, dass eine Beschädigung oder vorzeitige Ablösung der Wirkstoff-tragenden Beschichtung vor und/oder während der Implantation vermieden wird. Die Schutzschicht schützt die darunter liegenden Schichten und Bestandteile beispielsweise vor einer mechanischen Belastung. Dadurch, dass die Schutzschicht selbst biokorrodierbar ist, ist sichergestellt, dass die Schutzschicht nach erfolgter Implantation durch Biokorrosion schnell abgebaut wird und damit die Wirkstoff-tragende Beschichtung freigesetzt wird. Die Wirkstoff-tragende Beschichtung kann dann das ihr innewohnende Freisetzungsverhalten zeigen. Bei dem erfindungsgemäßen Stent ist also sichergestellt, dass sich die Wirkstoff-tragende Beschichtung nicht unkontrolliert vom Stent lösen kann. Somit kann am beabsichtigten Wirkort eine definierte Menge an Wirkstoff bereitgestellt und in gewünschter Weise freigesetzt werden.

Vorzugsweise weist der erfindungsgemäße Stent einen Grundkörper aus einem metallischen Implantatwerkstoff auf. Insbesondere kann der Grundkörper des Stents ganz oder in Teilen aus einem biokorrodierbaren metallischen Werkstoff bestehen oder diesen enthalten Insbesondere besteht der metallische Grundkörper aus Magnesium, einer biokorrodierbaren Magnesiumlegierung, Reineisen, einer biokorrodierbaren Eisenlegierung, einer biokorrodierbaren Wolframlegierung, einer biokorrodierbaren Zinklegierung oder einer biokorrodierbaren Molybdänlegierung. Der biokorrodierbare metallische Werkstoff ist besonders bevorzugt eine Magnesiumlegierung. Unter Magnesiumlegierung, Eisenlegierung, Zinklegierung, Molybdänlegierung oder Wolframlegierung wird vorliegend ein metallisches Gefüge verstanden, dessen Hauptkomponente Magnesium, Eisen, Zink, Molybdän oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Die Legierung ist so in ihrer Zusammensetzung zu wählen, dass sie biokorrodierbar ist.

Der erfindungsgemäße Stent weist auf Teilen oder der gesamten abluminalen Oberfläche des Grundkörpers eine Wirkstoff-freisetzende Beschichtung auf. Eine Beschichtung oder Schicht im Sinne der Erfindung ist eine zumindest abschnittsweise Auftragung der Komponenten der Beschichtung oder der Schicht auf den Grundkörper des Stents. Eine Dicke der Beschichtung oder Schicht liegt vorzugsweise im Bereich von 1 nm bis 100 µm, besonders bevorzugt 300 nm bis 15 µm. Die Wirkstoff-freisetzende Beschichtung kann direkt und bevorzugt ausschließlich auf Teile oder die gesamte abluminale Oberfläche des Grundkörpers aufgetragen werden. Zusätzlich können auch die Strutflanken (Strutseiten) eine Beschichtung tragen. Die Verarbeitung kann nach Standardverfahren für die Beschichtung erfolgen. Die Beschränkung der Wirkstoff-freisetzenden Beschichtung auf die abluminale Seite des Grundkörpers kann beispielsweise dadurch erreicht werden, dass die luminale Seite während des Auftragens durch Abschattung geschützt ist. Weitere abluminale Beschichtungen über Pipettierverfahren sind dem Fachmann bekannt. Es können einschichtige, aber auch mehrschichtige Systeme erstellt werden. Die Wirkstoff-freisetzende Beschichtung kann unmittelbar auf dem Grundkörper des Stents aufgebracht sein. Es können aber auch weitere zusätzliche ggf. haftvermittelnde Schichten dazwischen vorgesehen sein, wie beispielsweise eine oder mehrere Parylene-haltige Schichten.

Die Wirkstoff-freisetzende Beschichtung umfasst oder besteht aus einem Polymer und einem Wirkstoff mit antiproliferativer Wirkung. Unter dem Wirkstoff mit antiproliferativer Wirkung kann sowohl ein einzelner Wirkstoff als auch eine Mischung mehrerer verschiedener Wirkstoffe verstanden werden. Geeignete Wirkstoffe sind dem Fachmann bekannt. Neben dem Wirkstoff kann die Beschichtung beispielsweise noch weitere Verbindungen oder Hilfsstoffe aufweisen, wie z. B. Stoffe zur Kontrolle der Freisetzung, der Konservierung bzw. der Stabilisierung des Wirkstoffs. Vorteilhafterweise kann der Wirkstoff Sirolimus oder ein Derivat davon umfassen oder ein Taxan bzw. ein Salz davon. Insbesondere sind Wirkstoffe vorteilhaft, die auf mTOR wirken, sowie RAS Inhibitoren, insbesondere solche, die die RAS-Adhäsion verhindern. Für die Zwecke der vorliegenden Erfindung werden unter dem Begriff "Taxan" chemische Verbindungen verstanden, die ein Diterpenhaltiges Grundgerüst aufweisen und die eine zytotoxische oder zytostatische Aktivität aufweisen. Bevorzugte Taxane sind solche, die für die Krebstherapie verwendet werden und umfassen Paclitaxel, Docetaxel, Larotaxel, Ortataxel und/oder Tesetaxel, sowie Salze und/oder Derivate davon. Besonders bevorzugt handelt es sich bei dem Taxan um Paclitaxel bzw. Salze davon.

Die Wirkstoff-freisetzende Beschichtung umfasst ein nicht degradables oder vorzugsweise degradables Polymer. Dem Fachmann sind geeignete Polymere sowie Polymer-WirkstoffKombinationen mit einem geeigneten Freisetzungsprofil bekannt. In einer bevorzugten Ausführungsform umfasst das Polymer der Wirkstoff-freisetzenden Beschichtung ein Polymer oder Copolymer ausgewählt aus PLGA, PLLA, PVP/PVA-Copolymeren, insbesondere PVP/PVA-Copolymeren mit einem Gewichtsverhältnis von PVP zu PVA von 20:80 bis 70:30, bevorzugt mit einem Gewichtsverhältnis von PVP zu PVA von 20:80, Vinylacetat-Crotonsäure-Copolymere, ein Terpolymer bestehend aus den Monomeren Vinylacetat, Vinylpropionat und Crotonsäure, Methylvinylether-Maleinsäureanhydrid-Coplymere, Acrylat-Harze, Copolymere aus PVP und Dimethylaminoethylacrylat, sowie Reinpolymere aus den Monomeren der genannten Polymere oder Copolymere.

Die Oberfläche der luminalen Seite des Grundkörpers des erfindungsgemäßen Stents kann in Teilen oder ganz strukturiert sein, so dass beispielsweise die Endothelialisierung der luminalen Oberfläche nach erfolgtem Abbau der biokorrodierbaren Schutzschicht begünstigt und ggf. beschleunigt ist.

Der erfindungsgemäße Stent zeichnet sich dadurch aus, dass sowohl die luminale Oberfläche des Grundkörpers als auch die abluminale, die Wirkstoff-freisetzende Beschichtung tragende Oberfläche des Grundkörpers vollständig und formschlüssig mit einer biokorrodierbaren Schutzschicht bedeckt ist. Dabei bildet die biokorrodierbare Schutzschicht die oberste Schicht des erfindungsgemäßen Stents. Für die Zwecke der vorliegenden Erfindung wird unter der "obersten Schicht" die Schicht des Stents verstanden, die nach erfolgter Implantation entweder der Gefäßwand (abluminale Seite) oder dem fließenden Blut (luminale Seite) am nächsten kommt oder bevorzugt sogar in direkten Kontakt damit gebracht wird.

Die biokorrodierbare Schutzschicht bedeckt die luminalen und abluminalen Oberflächen des Grundkörpers des Stents formschlüssig. Unter einer formschlüssigen Bedeckung wird dabei verstanden, dass die biokorrodierbare Schutzschicht die luminale und abluminale Oberfläche des Stents im Wesentlichen gegen die Umwelt abgrenzt. Dabei besteht der Formschluss insbesondere in allen Richtungen der zur Zylinderachse des Stents senkrechten Ebenen. Die Schicht stellt eine mechanische Sicherung (Schutzschicht) dar.

Unter einer Biokorrodierbarkeit im Sinne der vorliegenden Erfindung wird dabei verstanden, dass die Schutzschicht nach erfolgter Implantation am Implantationsort aufgrund von biologischen oder biochemischen Wechselwirkungen mit der Umwelt reagiert und abgebaut wird.

Als biokorrodierbar im Sinne der Erfindung werden Schichten, Stoffe, Werkstoffe, Legierungen und Elemente bezeichnet, bei denen in physiologischer Umgebung ein Abbau/Umbau stattfindet, so dass ein aus dem Werkstoff bestehender Formkörper ganz oder zumindest überwiegend nicht mehr vorhanden ist. Als Prüfmedium zur Testung des Korrosionsverhaltens eines in Frage kommenden Werkstoffs dient beispielsweise künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Werkstoffe wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen. Ein Stoff wird beispielsweise dann als biokorrodierbar bezeichnet, wenn der Stoff in oben genanntem Test spätestens nach Ablauf von 6 Monaten zu mehr als 50% korrodiert oder umgesetzt ist.

In einer bevorzugten Ausführungsform ist die Schutzschicht innerhalb eines kurzen Zeitraums biokorrodierbar. Die schnelle Biokorrodierbarkeit der Schutzschicht sorgt dafür, dass die Wirkstoff-freisetzende Beschichtung nach erfolgter Implantation möglichst schnell zugänglich wird und damit das gewünschte Freisetzungsverhalten zeigen kann. Besonders bevorzugt zeichnet sich die Schutzschicht dadurch aus, dass diese innerhalb eines Zeitraums von 1 Tag bis 6 Monaten zu mindestens 50% korrodiert ist. Die zeitliche Abhängigkeiten zwischen degradabler Schutzschicht und degradablen Carrier werden wie folgt beschreiben (ta <= tb, tb <= 5 Jahre, vorzugsweise 6 Monate).

In einer weiteren bevorzugten Ausführungsform ist die biokorrodierbare Schutzschicht derart ausgeführt, dass diese durchlässig ist für den Wirkstoff der Wirkstoff-freisetzenden Beschichtung. Somit kann sichergestellt werden, dass auch während der Korrosionsphase der Schutzschicht bereits Wirkstoff freigesetzt werden kann und es somit nicht zu einer Verzögerung der Wirkstoffabgabe kommt.

Die biokorrodierbare Schutzschicht ist bevorzugt frei von Wirkstoffen. In einer besonderen Ausführungsform kann die biokorrodierbare Schutzschicht allerdings einen zweiten Wirkstoff enthalten, wobei dieser zweite Wirkstoff bevorzugt keine negative Wirkung (z.B. antiproliferative Wirkung) auf Endothelzellen hat. Bei dem zweiten Wirkstoff kann es sich insbesondere um einen Wirkstoff handeln, der die Einheilung des Stents nach erfolgter Implantation befördert.

Die biokorrodierbare Schutzschicht kann beispielsweise ein biokorrodierbares Polymer, ein biokorrodierbares Saccharid oder eine biokorrodierbare Gelatine enthalten oder daraus bestehen.

In einer bevorzugten Ausführungsform kann die biokorrodierbare Schutzschicht ein kurzkettiges PLGA Polymer mit 50 % bis 65 % PLA-Monomereinheiten und 35 % bis 50 % PGA-Monomereinheiten enthalten oder daraus bestehen. Die iv Werte betragen dabei 0,15 - 0,94 dl/g (in HFIP; 0,1 %; 25 °C) vorzugsweise 0,3 - 0,5 dL/g. entsprechend einer mittleren Molmasse von ca. 15.000 - 25.000 Da. Insbesondere kann ein kurzkettiges PLGA Polymer verwendet werden, welches die Monomere PLA und PGA in einem Gewichtsverhältnis von 50:50 und ein mittleres Molekulargewicht von 20.000 Da aufweist.

In einer anderen bevorzugten Ausführungsform kann die biokorrodierbare Schutzschicht hochbloomige Gelatine, mittelbloomige Gelatine oder eine Mischung daraus enthalten oder daraus bestehen. Bevorzugt machen die Gelatinebestandteile 1 bis 20 Gew.-% der biokorrodierbaren Schutzschicht aus, besonders bevorzugt 5 bis 10 Gew.-%, wobei sich die Angaben in Gew.-% auf das Gesamtgewicht der biokorrodierbaren Schutzschicht beziehen.

Ein Vorteil, der mit der Verwendung von Gelatine verbunden ist, ist dass eine Gelatine oder ein Gelatinegemisch verwendet werden kann, deren Erstarrungspunkt bei einer Temperatur zu liegen kommt, die unterhalb der Körpertemperatur liegt. Dadurch wird erreicht, dass die biokorrodierbare Schutzschicht solange intakt und belastbar bleibt, wie die Erstarrungstemperatur nicht überschritten wird. Nach erfolgter Implantation erwärmt sich der Stent und damit auch die Schutzschicht langsam auf Körpertemperatur, die Erstarrungstemperatur wird überschritten und die Gelatineschicht beginnt sich zu verflüssigen.

Die biokorrodierbare Schutzschicht kann ein Gelatinegemisch umfassen oder daraus bestehen, wobei die Gelatinebestandteile des Gelatinegemisches 10 - 90 Gew.% hochbloomiger Gelatine mit einer Gelierkraft von ≥ 250 Bloom und 90 - 10 Gew.% mittelbloomiger Gelatine mit einer Gelierkraft von ≥ 50 bis < 250 Bloom betragen, wobei sich die Angaben in Gew.% auf das Gesamtgewicht der Gelatinebestandteile des Gelatinegemisches beziehen. Bevorzugt ergeben die Gew.% hochbloomige Gelatine und die Gew.% mittelbloomige Gelatine in der Summe immer 100% des Gesamtgewichts der Gelatinebestandteile des Gelatinegemisches. Die hochbloomige Gelatine kann eine Gelierkraft von ≥ 250 bis 400 Bloom aufweisen, bevorzugt von ≥ 250 bis einschließlich 300 Bloom.

Dabei beschreibt das "Bloom" als Einheit die Gelierkraft von Gelatine. Die Kennzahl gibt die Masse in Gramm an, die benötigt wird, um einen Stempel von 0,5 Zoll Durchmesser die Oberfläche von 112 Gramm einer 6,67%-igen (w/w) Gelatine 4 mm tief verformen zu lassen. Dabei ist der Bloom-Wert höher, je höher die Gelierkraft der Gelatine ist. Die Bloom-Bestimmung findet standardisiert bei +10°C statt, wobei der zu prüfende Gelatineprüfling vorher für 17 Stunden bei +10°C gereift ist. Zur Bloom-Bestimmung kann ein Gelatometer nach Bloom verwendet werden.

Die Gelatinebestandteile des Gelatinegemisches der biokorrodierbaren Schutzschicht des erfindungsgemäßen Stents können beispielsweise 25 bis 75 Gew.% hochbloomige Gelatine und 75 bis 25 Gew.% mittelbloomiger Gelatine betragen, bevorzugt 40 bis 60 Gew.% hochbloomige Gelatine und 60 bis 40 Gew.% mittelbloomiger Gelatine, besonders bevorzugt 50 Gew.% hochbloomige Gelatine und 50 Gew.% mittelbloomige Gelatine, wobei sich die Angaben in Gew.% jeweils auf das Gesamtgewicht der Gelatinebestandteile des Gelatinegemisches beziehen.

Gelatine ist ein Gemisch von Polypeptiden, je nach Gewinnung mit Molmassen von ca. 13.500 bis 500.000 g/mol (bestimmt durch SDS-Gelelektrophorese oder Gelchromatographie), das durch eine mehr oder weniger weit geführte Hydrolyse von Collagen gewonnen wird. Die Aminosäure-Zusammensetzung entspricht weitgehend der des Collagens, aus dem sie gewonnen wurde, und umfasst mit Ausnahme des Tryptophans und Methionins alle essentiellen Aminosäuren; Leitaminosäure ist Hydroxyprolin. Gelatine enthält 84 bis 90 Gew.% Eiweiß und 2 bis 4 Gew.% Mineralstoffe; der Rest besteht aus Wasser. Gelatine ist geruchlos und praktisch farblos, unlöslich in Ethanol, Ethern und Ketonen, jedoch löslich in Ethylenglycol, Glycerol, Formamid und Essigsäure. Man unterscheidet zwei Herstellungsweisen: Das saure Verfahren für Gelatine des Typs A und das alkalische Verfahren für Gelatine des Typs B. Der Rohstoff für Gelatine Typ A (überwiegend Schweineschwarten) wird einem dreitätigen Aufschlussprozess unterworfen. Bei der Herstellung der Gelatine Typ B werden Rinderspalt (Mittelschicht zwischen Leder und der Unterhaut) bzw. Knochen 10 -20 Tage mit Alkali behandelt. Die Festigkeit der Gallerte wird mit einem Gelatometer (texture analyzer) bestimmt und als Bloomzahl angegeben. Der isoelektrische Punkt der Gelatine liegt bei pH 7,5 bis 9,3 (Typ A) bzw. 4,7 bis 5,2 (Typ B).

Gelatine kann durch Reaktion vor allem der Amino-Gruppen mit mono- oder polyfunktionellen Reagenzien wie Acylierungsmitteln, Aldehyden, Epoxiden, Halogen-Verbindungen, Cyanamid oder aktivierten ungesättigten Verbindungen chemisch modifiziert und in ihren Eigenschaften breit variiert werden. Die dadurch erhaltenen Gelatine-Derivate werden vorliegend von dem Begriff Gelatine umfasst.

Die erfindungsgemäß eingesetzte Gelatine ist hochgradig biokompatibel und biokorrodierbar. Die Verarbeitung kann nach Standardverfahren erfolgen.

Zur Verarbeitung wird die Gelatine durch Erwärmen, z. B. mittels Mikrowelle, verflüssigt und die Wirkstoffe werden suspendiert oder gelöst. Die Zugabe sollte vor dem Gelieren, d.h. Ausbilden eines Gels, insbesondere eines Hydrogels, erfolgen.

Das Ansetzten der Gelatine für die Beschichtung/Befüllung von Kavitäten kann in gepufferten Lösungen erfolgen. Diese Lösungen lassen sich besonders einfach verarbeiten. Der pH-Wert der Lösungen liegt vorzugsweise im Bereich von pH 5 bis pH 8, um eine Hydrolyse der Gelatine während der Verarbeitung zu vermeiden, was zu einer geringeren Gelfestigkeit führen würde.

Die Beschichtung der luminalen und abluminalen Oberfläche des Grundkörpers des erfindungsgemäßen Stents mit einer Gelatine enthaltenden, biokorrodierbaren Schutzschicht kann nach bekannten Verfahren erfolgen, wie beispielsweise das Aufbringen durch Sprühen, Tropfen, Tauchen, Kondensieren, Zerstäuben, Bedampfen und/oder Galvanisieren.

Insbesondere kann die das Gelatinegemisch enthaltende Schicht als Gel oder Hydrogel vorliegen.

Dabei kann die Gelschicht 1 bis 20 Gew.% Gelatinegemisch enthalten, bevorzugt 5 bis 10 Gew.%, wobei sich die Angaben in Gew.% auf das Gesamtgewicht der Gelschicht beziehen.

Figuren:
- Fig. 1: zeigt einen schematischen Querschnitt durch einen gestenteten Gefäßabschnitt
- Fig. 2: zeigt einen schematischen Querschnitt durch einen Strut eines erfindungsgemäßen Stents.

Nachfolgend wird die Erfindung anhand von Ausführungsbeispielen näher erläutert.

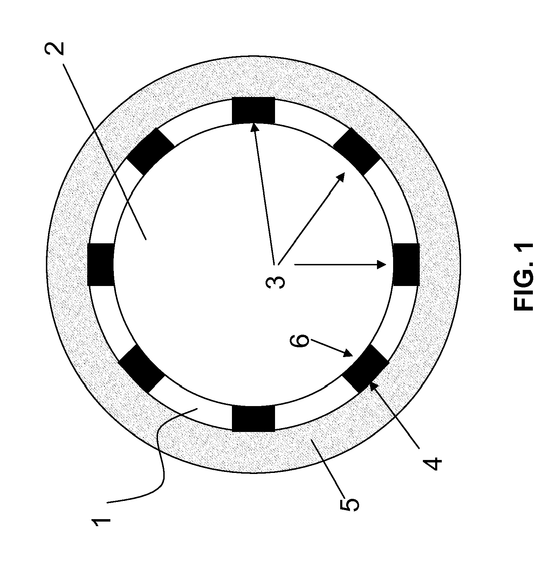

In Figur 1 ist ein schematischer Querschnitt durch einen Gefäßabschnitt, in den eine Ausführungsform des erfindungsgemäßen Stents implantiert wurde. Die Schnittebene liegt senkrecht zur Längsachse des Stents. Der erfindungsgemäße Stent 1 begrenzt ein Lumen 2 und besteht aus Struts 3, welche mit ihrer abluminalen Seite 4 die Gefäßwand 5 stützen und mit ihrer luminalen Seite 6 an das Lumen 2 grenzen.

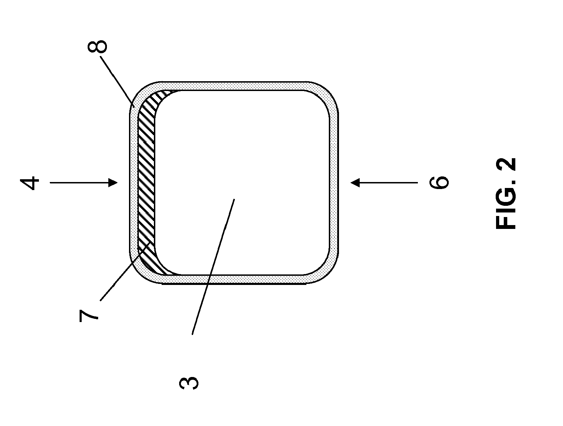

Figur 2 zeigt den Querschnitt durch einen Strut eines erfindungsgemäßen Stents. Auf der abluminalen Seite 4 weist der Strut 3 eine Wirkstoff-freisetzende Beschichtung 7 auf. Der Strut weist sowohl auf der luminalen Seite als auch auf der abluminalen, die Wirkstoff-freisetzende Beschichtung 7 tragenden Seite eine biokorrodierbare Schutzschicht 8 auf. Die biokorrodierbare Schutzschicht 8 bedeckt die luminale und die abluminale Oberfläche des Struts 3 vollständig und formschlüssig. Durch die Verwendung einer solchen biokorrodierbaren Schutzschicht 8 wird eine vorzeitige Ablösung von Teilen oder der gesamten Wirkstoff-freisetzenden Beschichtung 7 vom Strut 3 verhindert.

## Patentansprüche

1. Stent mit einem Grundkörper aus einem Implantatwerkstoff, wobei der Grundkörper auf der abluminalen Seite teilweise oder ganz mit einer Wirkstoff-freisetzenden Beschichtung bedeckt ist, die ein Polymer und einen Wirkstoff mit antiproliferativer Wirkung umfasst oder daraus besteht, **dadurch gekennzeichnet, dass** die luminale Oberfläche und die abluminale, die Wirkstoff-freisetzende Beschichtung tragende Oberfläche des Grundkörpers von einer biokorrodierbaren Schutzschicht formschlüssig bedeckt ist.

2. Stent nach Anspruch 1, **dadurch gekennzeichnet, dass** der Grundkörper die Wirkstoff-freisetzende Beschichtung ausschließlich auf der abluminalen Seite aufweist.

3. Stent nach Anspruch 1 oder 2, **dadurch gekennzeichnet, dass** der Grundkörper des Stent ganz oder in Teilen aus einem biokorrodierbaren metallischen Werkstoff besteht.

4. Stent nach Anspruch 3, **dadurch gekennzeichnet, dass** der biokorrodierbare metallische Werkstoff eine Magnesiumlegierung ist.

5. Stent nach einem der vorhergehenden Ansprüche, **dadurch gekennzeichnet, dass** nach erfolgter Implantation die biokorrodierbare Schutzschicht innerhalb eines Zeitraums von 1 Tag bis 6 Monate zu mindestens 50% korrodiert ist.

6. Stent nach einem der vorhergehenden Ansprüche, **dadurch gekennzeichnet, dass** die biokorrodierbare Schutzschicht durchlässig ist für den Wirkstoff der Wirkstoff-freisetzenden Beschichtung.

7. Stent nach einem der vorhergehenden Ansprüche, **dadurch gekennzeichnet, dass** die biokorrodierbare Schutzschicht ein biokorrodierbares Polymer oder ein biokorrodierbares Saccharid oder biokorrodierbare Gelatine enthält oder daraus besteht.

8. Stent nach einem der vorhergehenden Ansprüche, **dadurch gekennzeichnet, dass** die biokorrodierbare Schutzschicht ein kurzkettiges PLGA-Copolymer mit einem Gewichtsverhältnis von PLA zu PGA von 50:50 enthält oder daraus besteht.

9. Stent nach einem der Ansprüche 1 bis 7, **dadurch gekennzeichnet, dass** die biokorrodierbare Schutzschicht hochbloomige, mittelbloomige Gelatine oder eine Mischung daraus enthält oder daraus besteht.

10. Stent nach Anspruch 9, **dadurch gekennzeichnet, dass** die biokorrodierbare Schutzschicht ein Gelatinegemisch umfasst, wobei die Gelatinebestandteile des Gelatinegemisches aus 10 - 90 Gew.% hochbloomiger Gelatine mit einer Gelierkraft von ≥ 250 Bloom und 90 - 10 Gew.% mittelbloomiger Gelatine mit einer Gelierkraft von ≥ 50 bis < 250 Bloom besteht, wobei sich die Angaben in Gew.% auf das Gesamtgewicht der Gelatinebestandteile des Gelatinegemisches beziehen.

11. Stent nach Anspruch 10, **dadurch gekennzeichnet, dass** die hochbloomige Gelatine eine Gelierkraft von ≥ 250 bis 400 Bloom aufweist, bevorzugt von ≥ 250 bis 300 Bloom.

12. Stent nach einem der Ansprüche 10 oder 11, **dadurch gekennzeichnet, dass** die Gelatinebestandteile des Gelatinegemisches 25 bis 75 Gew-% hochbloomige Gelatine und 75 bis 25 Gew.% mittelbloomiger Gelatine betragen, bevorzugt 40 bis 60 Gew.% hochbloomige Gelatine und 60 bis 40 Gew.% mittelbloomiger Gelatine, besonders bevorzugt 50 Gew.% hochbloomige Gelatine und 50 Gew.% mittelbloomige Gelatine, wobei sich die Angaben in Gew.% auf das Gesamtgewicht der Gelatinebestandteile des Gelatinegemisches beziehen.

13. Stent nach einem der Ansprüche 10 bis 12, **dadurch gekennzeichnet, dass** das Gelatinegemisch 1 bis 20 Gew.% der biokorrodierbaren Schutzschicht ausmacht, bevorzugt 5 bis 10 Gew.%, wobei sich die Angaben in Gew.% auf das Gesamtgewicht der biokorrodierbaren Schutzschicht beziehen.

14. Stent nach einem der vorhergehenden Ansprüche, **dadurch gekennzeichnet, dass** die biokorrodierbare Schutzschicht einen zweiten Wirkstoff enthält, wobei der zweite Wirkstoff bevorzugt keine antiproliferative Wirkung auf Endothelzellen hat.

15. Stent nach einem der vorhergehenden Ansprüche, **dadurch gekennzeichnet, dass** sich zwischen der abluminalen Seite des Grundkörpers und der Wirkstoff-freisetzenden Beschichtung eine oder mehrere zusätzliche Schichten befinden, insbesondere eine ggf. haftvermittelnde Parylene-haltige Schicht.
